Europäisches Patentamt

European Patent Office

Office européen des brevets

(11) Publication number: **0 327 337**
**A2**

## EUROPEAN PATENT APPLICATION

(21) Application number: **89300963.9**

(22) Date of filing: **01.02.89**

(51) Int. Cl.⁴: **G 01 N 33/573**
G 01 N 33/72, G 01 N 33/76,
G 01 N 33/74, G 01 N 33/68,
G 01 N 33/577, C 12 P 21/00,
C 12 N 5/00
// C12N15/00, (C12P21/00,
C12R1:91)

(30) Priority: **01.02.88 US 150546    22.08.88 US 234974**
**06.01.89 US 294013**

(43) Date of publication of application:
**09.08.89   Bulletin   89/32**

(84) Designated Contracting States:
**AT BE CH DE ES FR GB GR IT LI LU NL SE**

(71) Applicant: **MONOCLONETICS INTERNATIONAL, INC.**
**18333 Egret Bay Boulevard Suite 270**
**Houston Texas 77058  (US)**

(72) Inventor: **Khan, Abas A.**
**5607 Dawnridge**
**Houston,Texas 77035  (US)**

**Warrington, Richard E.**
**18706 Capetown**
**Houston, Texas 77058  (US)**

(74) Representative: **Bizley, Richard Edward et al**
**BOULT, WADE & TENNANT 27 Furnival Street**
**London EC4A 1PQ  (GB)**

(54) Genetic markers for down's syndrome, fetal abnormalities and twins.

(57) Disclosed is an invention relating to detection of increased concentrations of SOD-1 in maternal body fluid as a screening test for fetal twins, and/or a high potential for spontaneous abortion or premature birth, or detecting SOD-1 concentrations in extracellular maternal body fluids as a screening test for fetal Trisomy 21 Down's syndrome. The invention also relates to detecting fetal Trisomy 21 Down's syndrome by screening maternal sera for increased concentrations of SOD-1 alone or SOD-1 and hemoglobin. The invention also relates to detecting an increased probability of fetal Trisomy 21 Down's syndrome by screening maternal sera for increased concentrations of SOD-1 alone or SOD-1 and hemoglobin, and one of more of the following: increased concentrations of hCG, decreased levels of unconjugated oestriol, and decreased levels of AFP. Other markers, such as maternal age and fetal femur length can also be used as indicators of fetal Trisomy 21 Down's syndrome.

**Description**

# GENETIC MARKERS FOR DOWN'S SYNDROME, FETAL ABNORMALITIES AND TWINS

The invention relates to the detection of twins, spontaneous abortion, premature birth, and abnormalities such as potential Down's syndrome progeny (Trisomy 21) by screening for increased concentrations of SOD-1 in maternal body fluid. The invention also relates to the detection of potential Down's syndrome progeny by screening for increased concentrations of SOD-1, or SOD-1 and hemoglobin, or, either SOD-1 alone or SOD-1 and hemoglobin together with one or more of the following: increased concentrations of human chorionic gonadotrophin ("hCG"), decreased levels of unconjugated oestriol, and decreased levels of alpha fetroprotein ("AFP"). In detecting Down's syndrome other parameters, such as maternal age and fetal femur length, are also useful.

Trisomy 21 (i.e., one additional 21st chromosome) accounts for 95 per cent of all cases of Down's syndrome. This condition is among the most common genetic defects, occurring in about 1 in every 800 births. Patients with this syndrome have characteristic faces, are mentally retarded, and at least 30 per cent have congenital heart disease.

Trisomy 21 occurs when homologous chromosomes fail to separate at anaphase, resulting in nondisjunction and the production of one monosomic and one trisomic daughter nuclei. In chromosomally normal women, the frequency of nondisjunction is age related. For women ages 20 to 30, the incidence increases linearly from about 0.3 per 1000 births to about 2 per 1000 births. After the age of 30, the incidence increases exponentially, at a rate of about 30 per cent per year. Women over 35 give birth to 35 per cent of all children with Trisomy 21 Down Syndrome.

Women under 20 also show an increasing incidence of Trisomy 21 Down's syndrome with falling age. Thus, a woman age 15 has about the same chance of having a Down's syndrome child as a woman between the ages of 30 and 35.

The risk for a woman under the age of 30 of having a second child with Trisomy 21 Down's syndrome is 1.4 per cent. Her risk after the age of 30 is 1 per cent plus the risk factor for a woman of that age.

The nondisjunction which results in Trisomy 21 Down's syndrome can also occur in the male. Nondisjunction in the male accounts for 20 to 25% of children with this syndrome. For fathers over the age of 55, the risk of having a Down's syndrome child may be about twice that expected after adjustment for the mother's age.

Prior to birth, genetic defects in the fetus (including Trisomy 21) can be detected through amniocentesis. In amniocentesis 10 to 20 ml of amniotic fluid are withdrawn from the amniotic cavity between the 15th and 16th week of pregnancy. The amniotic fluid cells are then grown in tissue culture for about two weeks. Thereafter, the cells are stained, and the chromosomes are counted to identify genetic abnormalities such as Trisomy 21.

Cell culturing is, however, an expensive, skilled labor-intensive, and time-consuming process, often costing up to $1200. Further, the cultures are easily destroyed if contamination enters. Thus, prior to cell culturing, the fluid is often screened for proteins which indicate the existence of certain defects (so-called "genetic markers.") If the screening is positive, cell culturing can then be done to verify the existence of the defect. If negative, the expense of culturing can be foregone. Screening is currently commonplace, because most pregnant women are now being advised to have amniotic fluid withdrawn and screened for the genetic marker AFP.

Elevated levels of AFP indicate open neural tube defects, such as anencephaly and Spina Bifida, as well as other fetal abnormalities. If AFP is monitored alone, however, it is not a particularly reliable screening test for Trisomy 21.

The current screening tests for Trisomy 21 are deficient. Further, because of the expense of the more accurate cell culturing technique, this test is often foregone by the patient. In view of the frequency with which Trisomy 21 occurs, markers (and a screening test based on such markers) which gave a truer indication of the presence of Trisomy 21 would clearly be desirable, particularly for women in the higher risk groups.

By 1981 it was recognized that Trisomy 21 patients showed increased concentrations of SOD-1 in Red Blood Cell lysates. See B.C. Del Villano & J.A. Tischfield, "Quantitation of Human Cuprozinc SOD-1 by Radioimmunoassay and Its Possible Significance in Disease," Immunoassay Methods at 366-67 (1981). However, there is no suggestion in this article to screen maternal body fluid for SOD-1 as a test for fetal Trisomy 21. The authors only state that this discovery could shed light on the role of SOD-1 in normal metabolism, or indicate the consequences of having excess SOD-1. They also indicate that further experiments could relate the SOD-1 level to the degree of retardation.

A 1985 study sought to determine whether increased concentrations of SOD-1 were to be found in Trisomy 21 fetal fibroblasts, amniotic cells and amniotic fluid. See M.A. Bateman, M.G. Mattei, A. Abret, M. Gamerre & J.F. Mattei, "Immunoreactive SOD-1 in Amniotic Fluid, Amniotic Cells and Fibroblasts from Trisomy 21 Fetus," Acta Poediatr. Scand. 74:697-700 (1985). The authors concluded that it was not possible to establish a relationship between SOD-1 levels in amniotic fluid and Trisomy 21 in the fetus. Id. at 699. Thus, a correlation between increased SOD-1 concentrations in maternal body fluids and fetal Trisomy 21 is a significant departure from the teachings of the prior art.

Similarly, detecting increased concentrations of SOD-1 in maternal body fluids and/or increased concentrations of hemoglobin has never been suggested as a screening test for Trisomy 21. Detecting increased concentrations of SOD-1 in maternal body fluids and/or increased concentrations of hemoglobin, together with one or more of increased concentrations of hCG, decreased levels of unconjugated oestriol, and decreased levels of AFP, has also never been suggested as a screening test for Trisomy 21.

A screening test for twins, spontaneous abortion, or premature birth has never been discovered. Such a test would allow parents and health care professionals to prepare, both emotionally and medically, for these occurrences.

The invention relates to detection of increased concentrations of SOD-1 in maternal body fluid as a screening test for fetal twins, and/or a high potential for spontaneous abortion or premature birth, or detecting SOD-1 concentrations in extracellular maternal body fluids as a screening test for fetal Trisomy 21 Down's syndrome. The invention also relates to detecting fetal Trisomy 21 Down's syndrome by screening maternal sera for increased concentrations of SOD-1 alone or both SOD-1 and hemoglobin. The invention also relates to detecting fetal Trisomy 21 Down's syndrome by screening maternal sera for increased concentrations of SOD-1 alone or both SOD-1 and hemoglobin, and one of more of the following: increased concentrations of hCG, decreased levels of unconjugated oestriol, and decreased levels of AFP.

To accomplish the screening for SOD-1 or SOD-1 and hemoglobin, one first assays body fluid samples which are know to be positive or negative for the aforementioned fetal conditions. One then arbitrarily determines a threshold minimum concentration. The threshold minimum concentration is a concentration which a high percentage of positive samples exceed, and which a high percentage of negative samples fall below. This concentration can, however, be adjusted. It can be raised to artificially reduce the number of false positive samples, at the expense of increasing the false negatives, or it can be lowered to reduce the false negatives, at the expense of increasing the false positives.

The unknown samples are then assayed for hemoglobin and/or SOD-1 to determine whether the indicator(s) is above the threshold. An elevated level indicates a positive result.

Such a screening test is relatively quick and simple to perform. If fetal Down's syndrome is under study and the test shows that the SOD-1 levels (or SOD-1 and hemoglobin levels) are above threshold, karyotyping can be done to confirm the result.

Essentially the same techniques can be used to detect the levels of hCG, unconjugated oestriol, or AFP. If hCG levels are elevated, or if unconjugated oestriol or AFP is depressed, it must be considered whether either or both hemoglobin levels or SOD-1 levels are also increased. If the total of some or all of these indicators points to a positive result, the indication of fetal Trisomy 21 may be stronger than when monitoring only hemoglobin and/or SOD-1.

It also may be desirable to monitor other parameters, such as maternal age and fetal femur length. These indicia of fetal Trisomy 21 Down's syndrome may also strengthen the indication of Trisomy 21.

It appears that a screening test for Trisomy 21 can be performed relatively early in the pregnancy; perhaps as early as the ninth week. The SOD-1 (and hemoglobin) levels are believed to show a sufficient increase at that time to serve as reliable indicators of Trisomy 21. Where hCG is used as an additional determinant, this could lower this date as far down as the eighth week. hCG levels are believed to show significant elevation by the eighth week for most mothers with Trisomy 21 fetuses.

The invention further relates to detecting increased concentrations of SOD-1 in maternal body fluids as a screening test for predisposition towards psychoses, rheumatoid arthritis, or alcoholism, and correlating SOD-1 concentrations with Alzheimer's disease, which is also known to be associated with Trisomy 21.

In the practice of the invention, concentrations are considered increased, or depressed, when they exceed, or fall below, an arbitrarily selected threshold. The threshold concentration is a concentration which a high percentage of positive samples indicate as positives, and which a high percentage of negative samples indicate as negatives. This concentration can, however, be adjusted. It can be changed one way or the other to artificially reduce the number of false positive samples, at the expense of increasing the false negatives, or to reduce the false negatives, at the expense of increasing the false positives. The particular threshold selected will vary depending on the type of sample assayed (i.e., serum or amniotic fluid) and depending on the gestational age of the sample.

Rather than setting threshold concentrations, one can also analyze the probability of fetal Down's syndrome at a particular maternal age for particular concentrations of SOD-1, hemoglobin, hCG, unconjugated oestriol, and AFP in maternal body fluids. This is accomplished by following a multi-step statistical analysis. This analysis eventually produces a table showing detection rate and false positive rate for fetal Trisomy 21 Down's syndrome at particular maternal ages and for particular concentrations of SOD-1, hemoglobin, hCG, unconjugated oestriol, and AFP. The details of this procedure are described below and can also be found in Cuckle et al., "Estimating a Woman's Risk of Having a Pregnancy Associated with Down's Syndrome Using her Age and Serum Alpha-Fetoprotein Level," 94 British Journal of Obstetrics and Gynaecology 387-402 May 1987), and in Wald et al. "Maternal Serum Screening for Down's Syndrome in Early Pregnancy," 297 British Medical Journal 883-887 (October 8, 1988) (hereinafter "Wald II").

Quantification of SOD-1, hemoglobin, hCG, unconjugated oestriol and AFP can be done with either a monoclonal or polyclonal antibody assay, using any standard assay technique. For example, one can use an Enzyme Linked Immunoassay ("ELISA"), a Radioimmunoassay ("RIA"), or a luminescence assay, all of which can be single or tandem (TM) antibody type, and all of which can be carried out in solid or liquid phase. See Vol. 74, Methods in Enzymology part C for general description of a luminescense assay; T.E. Koerdte, J.E. Butler, 83 Journal of Immunological Methods at 283-299 (1985) for general description of a solid phase RIA and solid phase ELISA; U.S. Patent No. 4,376,110 for general description of a Tandem (TM) type assay.

Polyclonal antibodies are best described as a variety of antibodies directed to a number of different epitopes. They are produced by immunizing a number of host animals with a particular immunogen and then collecting serum from each. The sera will contain antibodies to a variety of antigens, i.e., all those to which the animal has been exposed, as well as antibodies to the immunogen under study.

Sera from the animals which produce polyclonal antibodies with the highest specificity and affinity for the immunogen are selected for assay use. The selection can be done by a number of standard assay techniques, for example, a RIA, an immunofluorescence assay, Western Blot analysis, an ELISA, or histochemical staining.

Monoclonal antibodies, in contrast to polyclonals, are directed to one particular epitope. They are produced by hybridoma cells which have all been cloned from a single fused cell. All the clones are identical to the parent. Accordingly, all hybridomas of the same clone produce identical antibodies which bind to the same epitope.

A method making monoclonal antibodies was first described by Koehler and Milstein. See Milstein et al., 256 Nature 495-97 (1975); Koehler et al., 6 Eur J. Immunol., 511-19 (1976). In general terms, a host animal, usually a mouse, is immunized and then sacrificed. B-cells are then removed, usually from the spleen or other lymphoid tissues. The removed B-cells are fused with myeloma cells to form hybridomas. The hybridomas which produce antibodies against the immunizing antigen are isolated from the other hydridomas and cells. The selected hybridomas are then used to manufacture the desired monoclonal antibodies.

Monoclonal antibodies have certain advantages over polyclonals. The primary advantage is that they are all directed to a particular epitope and are, therefore, more specific. However, it must be noted that polyclonals can often function in an assay equally as well as monoclonals. A further advantage is that large quantities of monoclonal antibodies can be readily produced. If properly preserved, the hybridomas are essentially immortal, due to the fusion with myeloma cells, and they can reproduce almost endlessly. Monoclonal antibodies can be made by a number of conventional techniques. In general, after the host animal, usually a mouse, is immunized, it is sacrificed and B-cells taken from the spleen are fused with myeloma cells. The fusion is done using a fusion reagent, such as polyethylene glycol 4000. The hybridomas resulting from the fusion can then be resuspended, transferred to 96 well plates, and grown.

A number of modifications of the immunization, fusion, and screening steps, and the above-described method of antibody production, are possible. First, host animals other than mice can be used. Alternatively, human hybridomas could be made by withdrawing blood or tissue for in vitro immunization.

Further, reagents other than polyethylene glycol 4000 can be used for the fusion. In the alternative, the chemical fusion used in the invention can be replaced by electrical fusion. This technique is well established. Additionally, instead of fusion, one could also transform a B-cell to make it immortal using, for example, an Epstein Barr Virus. (For a method of transforming a B-cell, See "Continuously Proliferating Human Cell Lines Synthesizing Antibody of Predetermined Specificity," Zurawski, V.R. et al in Monoclonal Antibodies, ed. by Kennett R.H. et al, Plenum Press, N.Y. 1980, pp 19-33.)

In the present invention the hybridomas which produced the monoclonal antibodies to SOD-1 were isolated by a series of screening procedures. First, supernatants were taken from those wells in the 96-well plates which contained large numbers of clones. The wells containing clones producing antibody to SOD-1 were determined by adding radioactively labeled SOD-1 to each well, and determining the amount which became bound. The isolation could be determined with a number of other techniques, for example, by an ELISA, an immunofluorescence assay, Western Blot analysis, or an immunohistochemical staining. The only consideration is that the procedure selects for hybridomas which secrete monoclonal antibodies specific for SOD-1.

The cells selected were then limit diluted, grown, and characterized by a RIA procedure for specificity, sensitivity, and affinity.

T. Portsmann et al., in an article entitled "A Rapid and Sensitive Enzyme Immunoassay for Cu/Zn Superoxide Dismutase with Polyclonal and Monoclonal Antibodies," Vol. 171 Clinica Chemica Acta 1-10, describe monoclonal and polyclonal antibody based assays for SOD-1. These methods could also be used with the SOD-1 detection of the invention.

Polyclonal or monoclonal antibodies to hemoglobin, which can be also be used in detecting fetal Trisomy 21, can be prepared by essentially the same techniques described above.

Monoclonal antibodies to hCG are described in M. H. Bogart et al., "Abnormal Maternal Serum Chorionic Gonadotropin Levels in Pregnancies with Fetal Chromosome Abnormalities," 7 Prenatal Diagnosis 623-630 (1987). The monoclonals used by Bogart et al. are essentially of two types: one which detected alpha-HCG; and one which detected both intact hCG and free beta-HCG. When the latter antibody was used for detecting elevated levels of hCG (MoM), there was a greater correlation with Trisomy 21 fetuses, (using 2.5 MoM as the threshold) , than for the anti-alpha-HCG antibody. Based on these results, it is believed that the antibody which is to free beta-HCG and intact hCG is actually binding to a beta epitope on the intact hCG. It might also be that the alpha epitope on the intact hCG is somehow not available to react, which would explain why the anti-alpha-HCG antibody did not detect Trisomy 21 as reliably.

Unconjugated oestriol can be assayed as described in Wald et al. "Maternal Serum Unconjugated Oestriol as an Antenatal Screening Test for Down's Syndrome," 95 British Journal of Obstetrics and Gynaecology 334-41 (April 1988) (hereinafter "Wald I"), i.e., by using a direct non-extraction radioimmunoassay (Amerlex oestriol RIA kit, Amersham). Maternal serum AFP can also be assayed with the technique described in Wald I, i.e., with an immunoradiometric assay (Boots-Celltech Diagnostics Ltd.). Wald I express the level of AFP and unconjugated oestriol in multiples of the median (MoM).

After the desired monoclonal or polyclonal antibodies are isolated, the final step is to use them to assay the antigen under study. An RIA was used for detecting concentrations of both SOD-1 and hemoglobin. Bogart et al also used an RIA for detecting hCG levels. As noted above, assays for unconjugated oestriol and AFP are well known.

A RIA is a so-called "competitive" assay, wherein labeled antigen and unlabelled antigen are added to an antibody-containing supernatant, and the complex is precipitated. The unlabelled antigen displaces some of the labeled antigen from the antibody binding sites, and the extent of displacement is determined by measuring either the amount of bound labeled antigen, or the amount of free labeled antigen.

If a series of samples of known concentrations of unlabelled antigen are added together with a fixed quantity of labeled antigen, determining the amount of bound (or displaced) labeled antigen resulting after the addition of each sample will allow preparation of a standard curve of labeled antigen versus concentration. When a sample with an unknown concentration of unlabelled antigen is added to a system of bound labeled antigen, and the bound (or displaced) labeled antigen is determined, the standard curve can be used to determine the concentration of the system. It must be emphasized that rather than a competitive assay, or rather than a RIA, polyclonal (or monoclonal) antibodies could be used to determine SOD-1 or hemoglobin concentrations with any of the other aforementioned assay techniques.

Examples of the methods employed in producing the antibodies and conducting the assays of the invention will now be described in greater detail.

## Example of Making Monoclonal Antibodies

(i) Immunization: 8 BALB/C mice which were 4 to 6 weeks old were selected for immunization. SOD-1 was diluted in normal saline (0.9% NaCl) and then emulsified in Freunds complete adjuvant by drawing it in and out of a syringe. The final solution contained 100 to 200 micrograms of SOD-1 per 100 microliters, and 100 microliters was injected intradermally into different sites in each mouse. A booster injection of the same amount of SOD-1 at the same concentration was given after 4 to 6 weeks. This time, however, the SOD-1 was emulsified in incomplete Freunds adjuvant.

4-6 weeks after the booster was given, the mice were again immunized with the same amount and concentration of SOD-1 (mixed in normal saline) by intrasplenic injection. Four days later, the mice were sacrificed and their spleens were removed in preparation for the fusion.

(ii) Fusion for Hybridoma: From the spleens, single cell suspensions of spleen cells were prepared. Spleen tissue was diluted in Dulbecco's Modification of Eagles Basal Medium ("DMEM") and spun in a centrifuge at 1000g for 10 minutes. The supernatant was withdrawn, and sp2/0 myeloma cells maintained in culture were added to the cell pellet and resuspended.

The resultant suspension was then spun at 1000g for 10 minutes, and the supernatant was withdrawn. 1 ml of polyethylene glycol 4000 was then added gradually over 10 minutes, together with 20 ml of DMEM.

The fused cells were spun down, the supernatant was withdrawn, and the cells were resuspended in 100 ml of DMEM and added in aliquots of 100 microliters to each of 96 Wells of a 96 well plate. 10 plates were so prepared. The fusion products were grown in the plates for two weeks. See also K. Flurkey, M.B. Bolger & D.S. Linthicum, "Preparation and Characteristics of Antisera and Monoclonal Antibodies to Serotonergic and Dopaminergic Ligands," 8 J. Neuroimmunol. at 115-127 (1985) for a description of a hybridoma production procedure.

(iii) Initial Screening Process: The initial screening process was to pick up the SOD-1 antibody producing clones in the wells. 100 microliters of the hybridoma supernatant (some of which should contain the SOD-1 antibody) was transferred to 96 well polyvinyl chloride plates. The plates had previously coated with goat anti mouse IgG (which can be purchased from Miles Laboratories) at a concentration of 0.05 mg/ml. The plates were then incubated for 2 hours at 37° C. After washing the plates, $^{125}$I labeled SOD-1 (labeled as described by Villano & Tischfield supra at 363) was added. The labeled SOD-1 was diluted in borate buffer saline, 0.1% BSA until it displayed roughly 5000 counts per minute ("CPM") per 100 microliters.

After incubating for 2 hours at 37° C, the plates were washed twice and the wells were cut off with a hot michrome wire and transferred into plastic tubes for gamma spectroscopy.

The samples corresponding to the wells showing the highest CPM, and therefore the most antibody producing cells, were then limit diluted and re-screened to assure the presence of only one clone. The cells were then grown in larger plates.

After growing, 1-3 million of the cells were injected into the peritoneal cavities of a mouse which had been primed 1 week before with pristane. Fluid from the peritoneal cavity was withdrawn 8-10 days later, and the ascites fluid was collected. Different titres of the ascites fluid were made by dilution with borate buffer saline pH 8.2-8.4 with 0.1% BSA. Those titres which exhibited 30-50% binding of SOD-1 (as determined by the RIA technique described below) were then selected for further evaluation by a secondary screening procedure.

(iv) Secondary or RIA Screening Process. The secondary or RIA screening procedure was to characterize the antibodies in terms of their specificity, sensitivity, and affinity for SOD-1. 100 microliters of the selected titres together with 100 microliters of various concentrations of SOD-1 and 100 microliters of $^{125}$I labeled

SOD-1 displaying 25,000 CPM, was added into polyethylene tubes and incubated for 4 hours at room temperature. Goat anti mouse IgG, diluted 1:1, was then added with polyethylene glycol 6000, and incubated for 15 minutes after vortexing the tubes. The tubes were spun for 15 minutes at 2500g. The supernatant was decanted and the pellet was assessed for radioactivity.

This secondary screening determined whether addition of 30 ng/ml of unlabelled SOD-1 caused significant displacement of labeled SOD-1 from the antibody binding sites in a reasonable time (4 hours was considered optimal). In other words, this step was to select those monoclonal antibodies which had a greater affinity for the unlabelled antigen than for the labeled. To achieve this, the final CPMs in the pellets were compared with the counts in reference pellets. The reference pellets were produced by precipitation of goat anti-mouse IgG, antibody and $^{125}$I SOD-1 as described above, with the only difference being that no unlabelled SOD-1 was present. Only those samples of antibody which showed a significant reduction in CPM over the reference (and thus significant binding of unlabelled SOD-1) would be selected for use in determining SOD-1 levels in amniotic fluid. See also K.F. Miller, D.J. Bolt & R.A. Goldsby, "A Rapid Solution-Phase Screening Technique for Hybridoma Culture Supernatants Using Radiolabelled Antigen and a Solid-Phase Immunoabsorbent," 59 J. Immunol. Methods at 277-280 (1983) for description of an RIA screening process.

At first, it turned out that no monoclonal antibodies of optional sensitivity were obtained. Nevertheless, it must be emphasized that the monoclonal antibodies which were obtained would function adequately for determining SOD-1 levels in amniotic fluid, or other maternal body fluid, if a non-competitive assay such as an ELISA is used. However, polyclonals with a higher affinity for unlabeled SOD-1 were obtained by the process described below, and these were used for the SOD-1 assay.

Since performing these assays, further experimentation has led to isolation of a higher affinity monoclonal antibody which will function well in a competitive assay (in contrast to prior such antibodies). These monoclonal antibodies were isolated by essentially the same techniques described above. These monoclonal antibodies have been placed on deposit at the American Type Culture Collection under Access No. HB-9937. Such antibodies and corresponding hybridomas are part of the invention.

## Example of Making Polyclonal Antibodies

(i) Immunization: 8 BALB/C mice were immunized by essentially the same procedure as that described for the monoclonal. The only difference was that no final intrasplenic injection was given. 10-12 days after the booster injection was given, the blood was withdrawn, spun down, and the serum collected from each mouse. This serum, containing the polyclonal antibodies, was then screened essentially by the same screening processes described above.

(ii) RIA screening steps: Several different titres of sera from each mouse were made by diluting the sera with borate buffer saline pH 8.2-8.4, 0.1% BSA. The titres which exhibited 30-50% binding of SOD-1 (as determined by a RIA technique) were then selected. A 1:1000 titre of sera from 2 of the 8 mice was chosen.

These titres were then checked to determine whether 30 ng/ml of unlabelled SOD-1 caused significant displacement of labeled SOD-1 from the antibody binding sites within a four hour period. The results were satisfactory, and these titres were deemed acceptable for use in determining SOD-1 concentrations in amniotic fluid.

Because more sera is required for polyclonals than for monoclonals, a larger animal than a mouse is needed to produce polyclonals in commercial quantities. The same polyclonal immunization and selection procedure described above can also be carried out in larger animals, for example goats.

## Example of Using the Polyclonals in a Radioimmunoassay

Determination of SOD-1 levels in amniotic fluid was done with the 1:1000 polyclonal titre selected, and using an RIA. The RIA was done essentially as described above for the RIA screening procedure. This time, however, several various known concentrations of SOD-1 were mixed with the 1:1000 polyclonal titre and the labeled SOD-1. After the goat anti mouse IgG was added, the pellet was assessed for radioactivity. From using the samples with known concentrations of SOD-1, a standard curve was developed to be used in determining SOD-1 concentrations in unknown fluid samples. The procedure is described in greater detail below.

(i) Checking Procedure

The system must be checked to ensure proper binding. Labeled SOD-1 was added to a tube and the adjusted mean CPM was calculated. (Hereinafter "Total").

In calculating all values, duplicates of the tubes under consideration were made. The mean of the CPM of both tubes was calculated, and the non-specific binding counts (or background counts), were subtracted from the mean CPM to give the adjusted mean CPM. In another tube, SOD-1 antibody and goat anti-mouse IgG were then added, and the adjusted mean CPM of the pellet was calculated. (Hereinafter "$B_0$"). The $B_0$/Total x 100 should be 35% plus or minus 3%. It was found to be 34.8%.

The system is also checked to ensure that non-specific binding counts are within normal limits. An assay is run with all unlabelled SOD-1 and the adjusted mean CPM of the pellet is calculated. This value is designated "Blank." "Blank"/Total x 100 should be less than 10%. It was found to be 6.8%.

## (ii) RIA Procedure

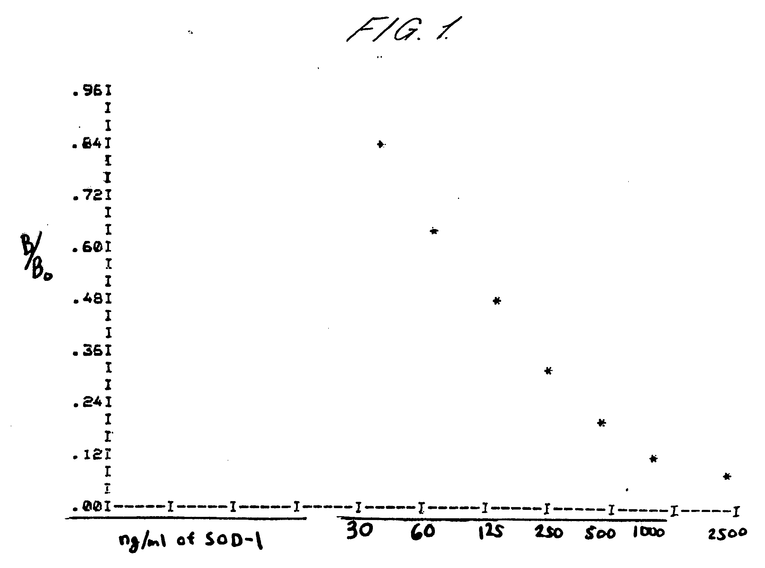

A RIA with seven standards containing known concentrations of SOD-1, ranging from 30 ng/ml to 2500 ng/ml, was then conducted. The adjusted mean CPM of the seven pellets was calculated (hereinafter "B"), and $B/B_0$ was plotted against the concentration of each sample to yield the standard curve shown in Figure 1 of the accompanying drawings.

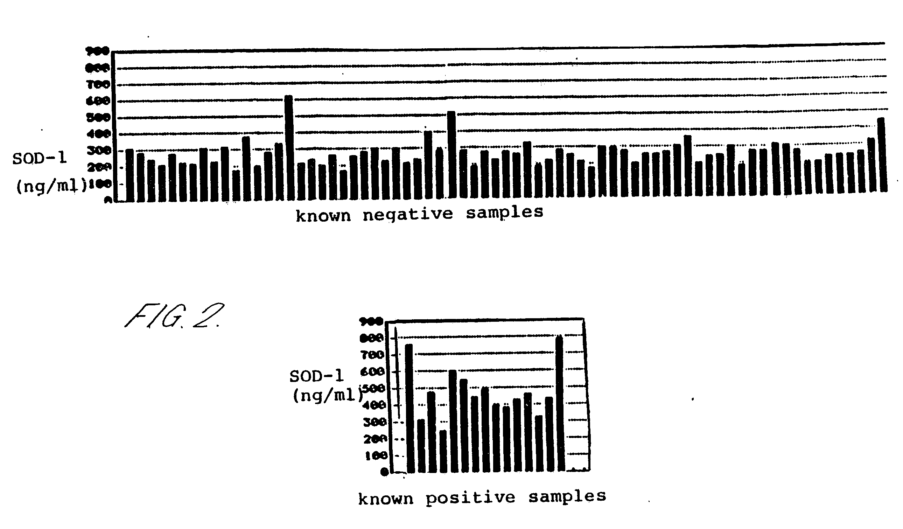

The RIA was then done with 71 different samples of amniotic fluid in which the fetus was known to be negative for Trisomy 21, and with 15 known positive samples, all of which were diluted 1:1. Sixty of the 86 samples tested were from women in the 13th to 20th week of gestation. For 25 samples, the gestational age was not known with certainty, although all were from at least the 12th week or later. One of the positive samples was from a woman in the 28th week of pregnancy. The standard curve was used to correlate the $B/B_0$ values obtained and determine the nanograms per ml of SOD-1 in the amniotic fluid. The results appear in bar graph form shown in Figure 2 of the accompanying drawings.

Of the 71 known negative samples, only 9 (or 13%) showed SOD-1 levels above 314 ng/ml. Thus, 87% of the known negatives were below the threshold. Of the 15 known positive samples, only 1 (or 7%) showed SOD-1 levels below 314 ng/ml. Thus, 93% of the known positives were above the threshold level. Due to the minimization of false positives and false negatives at the 314 ng/ml level, this concentration was selected as the threshold for indication of Trisomy 21 in amniotic fluid. These results show that the assay is suitable as a screening test for determining which patients should undergo karyotyping.

The mothers tested ranged in age from 20 to 42 years. Age did not seem to affect the threshold or the reliability of the test as an indicator of fetal Trisomy 21.

## Screening Maternal Sera for SOD-1

A similar screening process, using RIA techniques, was also performed on sera from 176 mothers (in at least the twelfth week of gestation) who carried a normal fetus, and sera from 21 mothers who carried a fetus with Trisomy 21. The gestational ages were all between the 15th and 18th weeks. The ages of all mothers carrying a Trisomy 21 fetus were between 35 and 43 years. Because sera, rather than amniotic fluid was being screened, a different threshold was needed.

Of the known positive samples, 71% showed SOD-1 levels above 105 ng/ml, and only 29% were "false negatives," i.e., below this level. Of the negative samples, 85% were below 105 ng/ml, and only 15% were "false positives," i.e., above this level. Thus, due to the optimization of false positives and negatives at 105 ng/ml, this concentration was selected as the threshold minimum concentration. It can be seen that elevated SOD-1 concentration in maternal sera is also a reliable indicator of Trisomy 21, even though the threshold is different than for amniotic fluid.

The threshold concentration can be adjusted to accomplish various purposes. If it is increased first to 115 and then 125 ng/ml, the false positives decline to 10 and 8%, respectively. However, at both of these higher thresholds, the false negatives increase to 42%, making the test a less reliable indicator of fetal Trisomy 21. Notwithstanding these results, some adjustment to the threshold is to be expected in further refining the test.

## SOD-1 and Hemoglobin Screening

The serum samples which were tested for SOD-1 concentration were also tested for hemoglobin concentration. The hemoglobin concentration and SOD-1 concentration were then examined both together, and separately, as indicators for fetal Trisomy 21.

The hemoglobin concentration was determined using a polyclonal antibody RIA. In this RIA, after the screening steps were performed, a standard curve was constructed using samples with concentrations of 2, 10, 50, 500, and 2500 micrograms/ml of unlabelled hemoglobin in 0.1% BSA/PBS (0.10M). 25 microliters of each sample was added to tubes containing 100 microliters of rabbit polyclonal antibody (purchased from Accurate Chemical and Scientific Corp.) at a 1:200 titre, and 100 microliters of $^{125}$I labeled hemoglobin displaying 30,000 CPM (labeled as described by Villano & Tischfield, supra at 363). After addition of the sample, the tubes were incubated at room temperature for two hours, precipitated with goat anti-rabbit IgG and polyethylene glycol, spun, decanted, and monitored for CPMs. As described above for the SOD-1 RIA, duplicates of all tubes were made and the adjusted CPM was calculated.

The standard curve prepared by this procedure was then used to determine concentration of hemoglobin in unknown maternal sera samples, using the RIA procedure described above. Nevertheless, it is noted that hemoglobin concentration could also be measured with any assay technique or any technique sensitive enough to quantify microgram concentrations.

After monitoring the results, threshold concentrations of 85 micrograms/ml for hemoglobin, and 113 ng/ml for SOD-1, were selected. Only 5% of the 176 known negative samples were above these levels. This is a significant reduction in "false positives" from the 15% experienced when monitoring SOD-1 concentration alone.

There was also a slight reduction in "false negatives" with these thresholds. When monitoring SOD-1 alone, 29% of the positive samples were false negatives, whereas slightly more than 26% were false negatives when hemoglobin and SOD-1 were both monitored. These results indicate that measuring both SOD-1 and hemoglobin concentration in sera is a more reliable test for fetal Trisomy 21 than is monitoring SOD-1 concentration alone.

7

## hCG Screening with SOD-1 or SOD-1 and Hemoglobin

Using the techniques described by Bogart et al., supra, one can screen maternal serum (or other body fluids) for hCG. The antibody used would be the one mentioned above which binds both free beta hCG and intact hCG. Using 2.5 MoM as the minimum hCG level considered elevated, this screen could improve the results of the SOD-1 and/or hemoglobin screening tests. It would be used in conjunction with an SOD-1 and/or hemoglobin screening test. When there is increased concentration of SOD-1 alone or SOD-1 and hemoglobin as well as hCG, this would indicate a greater chance of a Trisomy 21 fetus. Such monitoring of the hCG level would also probably reduce the "false positives" experienced when monitoring only SOD-1 or SOD-1 and hemoglobin.

## Screening for SOD-1, or SOD-1 and Hemoglobin, with One or More of hCG, Unconjugated Oestriol, and AFP

Using the techniques described in Wald II, and well known to those skilled in the art, an assay for unconjugated oestriol and AFP in serum is designed. A series of serum samples, some known to be positive and some known to be negative, are assayed. From these results, and from the results from assays for hCG, SOD-1 and hemoglobin, a table is constructed. The table shows the false positive rate (proportion of unaffected pregnancies with positive results) for various "detection rates" (proportion of Down's syndrome pregnancies with positive results), when the variables of maternal age and concentrations of AFP, unconjugated oestriol, and hCG are considered separately and in various combinations.

The method of Wald II involves a multi-step analysis of the variables of maternal age and concentrations of AFP, unconjugated oestriol, and hCG. Through a statistical analysis, one eventually arrives at the aforementioned table. See Table III below.

The method of arriving at the results shown in Table III involved, first, determining for each year of maternal age at delivery, from 15 to 50, an estimate of the Down's syndrome risk. The precise method by which the results of eight survey were combined to yield a risk estimate for each age are described in detail in Cuckle et al., "Estimating a Woman's Risk of Having a Pregnancy Associated with Down's Syndrome Using her Age and Serum Alpha-Fetoprotein Level," 94 British Journal of Obstetrics and Gynaecology 387-402 (May 1987) (hereinafter "Cuckle et al."). In brief, Cuckle et al. took the weighted average of the separate risk estimates from each study, on a log scale, and each was weighted in proportion to the inverse of its variance. To reduce the random error associated with the risk estimate at each age, Cuckle et al. used a regression analysis of the probability of a birth with Down's syndrome on age using the constant plus exponential function of age model described by Lamson & Hook (1981). See Cuckle et al. at 388.

To estimate the Down's syndrome risk for a given age and AFP level, Cuckle et al multiplied the left-hand side of the age-specific odds ratio (Down's syndrome: unaffected pregnancy) by a factor known as the likelihood ratio. The likelihood ratio is the proportion of Down's syndrome pregnancies with the given AFP level divided by the proportion of unaffected pregnancies with the same level. Cuckle et al describe a method for estimating this ratio in situations where there are fewer than 1,000 affected pregnancies under study. See p. 388-89.

Cuckle et al then arbitrarily assigned six different risk level policies, each representing the risk of a Down's syndrome fetus in an individual woman.

Cuckle et al next determined maternal age-specific AFP cut-off levels, so that the risk of Down's syndrome in an individual woman with an AFP value at each cut-off level was one of: 1:100, 1:150, 1:200, 1:250, 1:300, and 1:350. The cut-off level of AFP for each risk level was determined by calculating the likelihood ratios needed to yield the specified risk from the age-specific risk (e.g., if the age-specific risk is 1:375 then the likelihood ratio for a 1:200 risk is 375/200). Using the likelihood ratio, one then solves an equation shown in Cuckle's Appendix 1 (p. 400) to determine the AFP cut-off level.

Cuckle et al also provide a method for estimating the detection rate and false positive rate at each risk level for Down's syndrome. However, these rates can also be derived from an examination of the data, without estimating, once one establishes the AFP cut-off levels mentioned above.

In Wald I, in addition to monitoring maternal age and AFP level, unconjugated oestriol was monitored. Essentially the method of Cuckle et al. was used to determine detection rate and false positive rate. However, an estimated correction for long-term between-batch assay variance for unconjugated oestriol was added. Further, the estimate of detection and false positive rates for all three variables was made with a fitted Gaussian frequency distribution, rather than the observed distribution used by Cuckle et al. The false positive rate and detection rate, using all three variables, is shown below in Table I

TABLE I

False Positive Rate

| Detection Rate | Age, AFP and Unconjugated Oestriol |
|---|---|
| 60 | 12 |
| 55 | 9.1 |
| 50 | 7.0 |
| 45 | 5.3 |
| 40 | 3.9 |
| 35 | 2.8 |
| 30 | 1.9 |
| 25 | 1.2 |
| 20 | 0.7 |

Wald II added the step of screening for the variable hCG. The hCG screening was done with an immunoradiometric assay (MAIA-clone kit calibrated to the first international reference preparation, manufactured by Serono). An estimate of the long term batch assay variance was obtained and was added to the estimate of the observed variances of maternal serum hCG to yield estimates of the standard deviation found in routine practice.

Wald II then calculated the detection rate and false positive rate using maternal age together with various combinations of concentrations of hCG, AFP, and unconjugated oestriol. The results are shown below in Table II.

## TABLE II

### False Positive Rate (%) for Age with*:

| Dectection Rate (%) | AFP | U.O.** | hCG | AFP, U.O. | AFP, hCG | U.O., hCG | AFP, U.O., hCG |
|---|---|---|---|---|---|---|---|
| 80 | 44 | 34 | 27 | 29 | 20 | 20 | 16 |
| 75 | 37 | 27 | 21 | 23 | 15 | 15 | 12 |
| 70 | 30 | 22 | 16 | 18 | 12 | 11 | 8.6 |
| 65 | 25 | 18 | 12 | 15 | 8.8 | 8.1 | 6.4 |
| 60 | 20 | 14 | 9.5 | 12 | 6.7 | 6.0 | 4.7 |
| 55 | 16 | 11 | 7.2 | 9.1 | 5.0 | 4.4 | 3.4 |
| 50 | 12 | 8.8 | 5.4 | 7.0 | 3.7 | 3.2 | 2.5 |
| 45 | 9.8 | 6.7 | 3.9 | 5.3 | 2.7 | 2.3 | 1.7 |
| 40 | 7.3 | 5.0 | 2.8 | 3.9 | 1.9 | 1.6 | 1.2 |
| 35 | 5.3 | 3.7 | 1.9 | 2.8 | 1.3 | 1.0 | 0.8 |
| 30 | 3.6 | 2.5 | 1.2 | 1.9 | 0.8 | 0.7 | 0.5 |
| 25 | 2.2 | 1.6 | 0.8 | 1.2 | 0.5 | 0.4 | 0.3 |
| 20 | 1.3 | 0.9 | 0.4 | 0.7 | 0.3 | 0.2 | 0.2 |

*Result is positive if risk of Down's syndrome is high when estimated from maternal age and results of biochemical test or tests.
**U.O. is unconjugated oestriol.

EP 0 327 337 A2

In the invention, the method of Wald I and Wald II is used to determine detection rate and false positive rate for maternal age and one or more of the antigens hCG, unconjugated oestriol, and AFP. In addition, however, SOD-1 and hemoglobin, or SOD-1, are added variables in all cases.

A table for "detection rate" against "false positives" is constructed as described above. This table has 21 columns (three times the number of columns in Table III). Each column represents a different combination of SOD-1, or SOD-1 and hemoglobin, and one or more of the antigens hCG, unconjugated oestriol, and AFP. The columns which represent a combinations of most or all of the variables (SOD-1, hemoglobin, hCG, unconjugated oestriol and AFP) should provide a detection rate and false positive rate considerably better than achieved in Wald II.

As explained above, using SOD-1 in maternal serum alone at a detection rate 71% there were 15% "false positives" at a cut-off level of 105 ng/ml. When monitoring both SOD-1 and hemoglobin in maternal serum, at a detection rate of 74% there were only 5% false positives, using cut-off levels of 85 micrograms/ml for hemoglobin and 113 ng/ml for SOD-1. In comparison, when monitoring the three variables AFP, hCG, and unconjugated oestriol, at a 70% detection rate there was an 8.6% false positive rate. When these SOD-1 and hemoglobin levels are combined by multi-variate analysis with the three variables monitored in Wald II, the detection rate should increase and the false positives should decrease. The combination of these variables should be a very effective screening test for fetal Trisomy 21 Down's syndrome, approaching 100% reliability.

Wald II also teaches that as a complementary technique to serum screening, one can measure fetal femur length with ultrasound screening. This could further improve the results of the screening tests discussed above.

## SOD-1 Screening for Twins and Probable Spontaneous Abortion

Amniotic fluid from two mothers who carried twins which had spontaneously aborted was assayed. These samples showed levels of 377 ng/ml and 412 ng/ml for SOD-1 -- well above the threshold level for Down Syndrome of 314 ng/ml. This demonstrates that elevated levels of SOD-1 in body fluids indicate twins and/or a high probability of spontaneous abortion. However, as noted above, the elevated SOD-1 concentrations which were found to be associated with twins or spontaneous abortion could also indicate fetal Trisomy 21. Because of the uncertainty associated with elevated SOD-1 levels, karyotyping is preferably performed on mothers with elevated levels as a conclusive test for Trisomy 21.

## SOD-1 Screening for Probable Premature Birth

A SOD-1 assay was also conducted on serum from a mother who carried a fetus which had been prematurely born.

A fairly high level of SOD-1 is present in normal serum. This is because SOD-1 is contained in erythrocytes and enters serum from the erythrocytes. The level of SOD-1 in serum can, therefore, vary considerably depending on the number of erythrocytes present in the sample. In order to correct for the effect of erythrocyte-source SOD-1, the concentration of hemoglobin (in micrograms/ml) -- which also originates in the erythrocytes -- was divided by the concentration of SOD-1 (in nanograms/ml).

Serum from a mother carrying a normal fetus showed a ratio of 1/1.13, whereas the mother carrying the prematurely born fetus had a ratio of 1/2.7. This indicates that elevated serum SOD-1 is an indicator of a high probability of premature birth. Once again, because elevated SOD-1 can also indicate fetal Trisomy 21, it is important to do karyotyping on all mothers which show elevated concentrations of SOD-1.

It should be understood that the description and examples above are exemplary only and not limiting, and that the scope of protection is defined in the claims which follow, and includes all equivalents of the subject matter of the claims.

## Claims

1. A method for detecting fetal Trisomy 21, comprising either:
   (a) measuring the concentration of SOD-1 in extracellular maternal body fluid; and
   determining whether said concentration is above a specified threshold for a woman at the same gestational period; or
   (b) measuring the concentration of SOD-1 and hemoglobin, in maternal serum; and
   determining wehther both said concentrations are above respective specified thresholds for a woman at the same gestational period; and optionally further including measuring the concentration of one or more of the determinants human chorionic gonodotrophin (hCG), unconjugated oestriol, and alpha fetoprotein; and
   determining whether the concentration of hCG is elevated above the median for a woman of a similar age and gestational period, and whether unconjugated oestriol and alpha fetoprotein are depressed below the median for a woman of similar age and gestastional period; or
   (c) measuring the concentration of hCG and SOD-1 alone or both hemoglobin and SOD-1 in maternal body fluid; and
   determining, optionally by assay, whether the hCG concentration is elevated andwhether the SOD-1 concentration alone or the concentrations of hemoglobin and SOD-1 are above a threshold minimum for a fetus of similar gestational period.

2. The method of claim 1 wherein in (a) either the fluid sample is maternal serum, and the test indicates a positive result when the concentration of SOD-1 is greater than 113 ng/ml for a woman in the twelfth to twentieth week of gestation, or in (a) the fluid smaple is amniotic fluid, and the test indicates a positive result when the concentration of SOD-1 is greater that 314 ng/ml.

3. The method of claim 1 wherein in (b) the SOD-1 threshold is 113 ng/ml and the hemoglobin threshold is 85 micrograms/ml for a woman in the twelfth to twentieth week of gestation.

4. The method of claim 1 wherein in (c) the body fluid is serum, hCG is considered elevated if it exceeds 2.5 MoM, and the minimum threshold for SOD-1 and hemoglobin is 113 ng/ml and 85 micrograms/ml, respectively.

5. A method for detecting a high probability of spontaneous abortion, premature birth, or twins, comprising:

measuring the concentration of SOD-1 in a sample of maternal body fluid, optionally amniotic fluid or serum; and

determining whether said concentration is above a specified threshold for an unaffected fetus of similar gestational period.

6. The method of claim 5 wherein either (a) the body fluid is amniotic fluid, a high probability of spontaneous abortion or twins is detected, and the threshold minimum concentration is substantially 314 ng/ml of SOD-1; or (b) the body fluid is serum and premature birth is being detected, and the ratio of serum hemoglobin concentration (micrograms/ml)over SOD-1 concentration (manograms/ml) is less than 1/1.13.

7. The method of claim 5 or claim 6 wherein the concentration of SOD-1 is assayed by an assay which is homogeneous or heterogeneous, one or two site immunoassay, employing monoclonal or polyclonal antibodies.

8. A method for screening for a high probability of premature birth comprising:

assaying a sample of maternal serum to detect the concentration of SOD-1 and of serum hemoglobin;

dividing said concentration of hemoglobin by the concentration of serum SOD-1; and

determining whether said ratio is below a specified threshold.

9. The method of claim 8 wherein the hemoglobin concentration is measured in micrograms/ml, the SOD-1 concentration is measured in nanograms/ml, and the threshold is $1/1.13 \times 10^3$.

10. A method for detecting the likelihood of fetal Trisomy 21 Down's syndrome, comprising:

assaying a series of samples of maternal sera from women of known age for the variables SOD-1 alone or SOD-1 and hemoglobin, as well as one or more of the variables hCG, unconjugated oestriol, and alpha fetoprotein, some of said samples being known to be positive and some negative for fetal Trisomy 21 Down's syndrome;

using the data to construct a table which correlates the proportion of Down's syndrome pregnancies with positive results and the proportion of unaffected pregnancies with positive results for particular concentrations of each variable assayed and for particular maternal ages;

measuring, in an unknown maternal serum sample, the concentration of the variables SOD-1 alone or SOD-1 and hemoglobin, as well as one or more of the variables hCG, unconjugated oestriol, and alpha fetoprotein;

determining, from the table, the probability that the unknown sample indicates fetal Trisomy 21 Down's syndrome.

11. The method of claim 10 wherein the variables assayed are SOD-1 and optionally hemoglobin, in either case as well as at least one of alpha fetoprotein, hCG, and unconjugated oestriol.

12. A method for detecting the likelihood of fetal Trisomy 21, comprising:

determining, for a number of different maternal ages at delivery, the risk of fetal Trisomy 21 Down's syndrome;

assaying a series of samples of maternal sera from women of known age for either or both of the variables SOD-1 and hemoglobin, as well as one or more of the variables hCG, unconjugated oestriol, and alpha fetoprotein, some of said samples being known to be positive and some negative for fetal Trisomy 21 Down's syndrome;

estimating the Down's syndrome risk for a given age and level of assayed variables;

determining a series of maternal age-specific cut-off levels for the variables assayed, so that the risk of Down's syndrome in an individual woman at each cut-off level is a specified proportion;

determining the proportion of Down's syndrome pregnancies with positive results and the proportion of unaffected pregnancies with positive results for particular concentrations of each variable assayed and for particular maternal ages;

constructing a table which correlates the proportion of Down's syndrome pregnancies with positive results and the proportion of unaffected pregnancies with positive results for particular concentrations of each variable assayed and for particular maternal ages;

measuring in an unknown maternal serum sample, the concentration of the variables SOD-1 alone or SOD-1 and hemoglobin, as well as one or more of the variables hCG, unconjugated oestriol, and alpha fetoprotein;

determining, from the table, the probability that the unknown sample indicates fetal Trisomy 21 Down's syndrome.

13. The method of claim 12 wherein the variables assayed are SOD-1,hemoglobin, alpha fetoprotein, hCG, and unconjugated oestriol, or SOD-1, alpha fetoprotein, hCG, and unconjugated oestriol.

14. The method of any one of claims 10 to 13 further including the step of measuring fetal femur length and determining whether said length is below the median, as an additional variable in screening for fetal Trisomy 21 Down's syndrome.

15. The method of any one of claims 10 to 14 wherein the particular concentrations of the variables assays is adjusted so that, in progressive increments of 5% starting from 20% and progressing to 80%, said percentages of the Trisomy 21 Down's syndrome pregnancies display positive results.

16. A monoclonal antibody to SOD-1 which has a high enough affinity to permit its use in a competitive assay involving the determination of an SOD-1 level.

17. A monoclonal antibody as defined in claim 16 and as obtainable from ATCC under No HB-9937.

18. A hybridoma capable of producing an antibody as defined in claim 16.

19. A hybridoma obtainable from ATCC under No. HB-9937.

FIG. 1

EP 0 327 337 A2

SOD-1
(ng/ml)

known negative samples

FIG. 2.

SOD-1
(ng/ml)

known positive samples

EP 0 327 337 A2